# EUROPEAN PATENT APPLICATION

(11) **EP 1 498 126 A1**
(43) Date of publication of application: **19.01.2005**
(21) Application number: 03717540.3
(22) Date of filing: 03.04.2003
(51) Int. Cl.: A61K 31/495, A61K 45/00, A61P 25/00, A61P 25/02, A61P 25/04, A61P 25/14, A61P 43/00, C07D 295/22

(54) **NEUROTROPHIC FACTOR PRODUCTION ACCELERATOR**

(30) Priority: 10.04.2002 JP 2002108552
(71) Applicant: FUJISAWA PHARMACEUTICAL CO., LTD., Osaka-shi Osaka 541-8514 (JP)
(72) Inventor: MATSUDA, Toshio, Settsu-shi, Osaka 566-0011 (JP); BABA, Akemichi, Nishinomiya-shi, Hyogo 662-0825 (JP); KOYAMA, Yutaka, Suita-shi, Osaka 565-0824 (JP)
(74) Representative: Gille Hrabal Struck Neidlein Prop Roos
(86) International application number: PCT/JP2003/004257
(87) International publication number: WO 2003/084542

(57) **Abstract**

The present invention relates to an a neurotrophic factor production accelerator comprising, as an active ingredient, a compound represented by the formula (I) below: 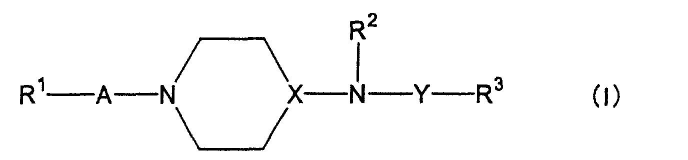 wherein R¹ is lower alkyl, aryl, ar(lower)alkoxy, or a heterocyclic group, and the above groups may be substituted with halogen, R² is a hydrogen atom or lower alkyl, R³ is cyclo(lower)alkyl, aryl or ar(lower)alkyl, and the above groups may be substituted with halogen, A is -CO-, -SO₂- or lower alkylene, X is N or CH, Y is -CO-, -SO₂- or -CONH-, a salt, a prodrug or a solvate thereof. According to the present invention, a neurotrophic factor production accelerator, which is useful for the prophylaxis or treatment of a motor nervous system or peripheral nervous system disease, can be provided.

SEQUENCE LISTING
<110> Fujisawa Pharmaceutical Corporation Ltd.

<120> Neurotrophic Factor Expression-promoting Agent

<130> 09543

<150> JP 2002-108552
<151> 2002-04-10

<160> 4

<170> PatentIn version 3.0

<210> 1
<211> 24
<212> DNA
<213> Artificial Sequ ence
<220>
<223> Oligonucleotide designed to act as PCR primer for detecting of bet
a-actin gene
<400> 1
gatggtgggt atgggtcaga agga 24

<210> 2
<211> 24
<212> DNA
<213> Artificial Sequence
<220>

<223> Oligonucleotide designed to  act as PCR primer for detection of bet
a-actin gene
<400> 2
gctcattgcc gatagtgatg acct                                    24

<210> 3
<211> 20
<212> DNA
<213> Artificial Sequence
<220>
<223> Oligonucleotide designed to act as PCR primer for detection of gli
al cell-line derived neurotrophic factor (GDNF) gene
<400> 3
atgaagttat gggatgtcgt                                         20

<210> 4
<211> 20
<212> DNA
<213> Artificial Sequence
<220>
<223> Oligonucleotide designed to act as PCR primer for detection of gli
al cell-line derived neurotrophic factor (GDNF) gene
<400> 4
cagggtcaga tacatccaca                                         20

## Description

### Technical Field

The present invention relates to a neurotrophic factor production accelerator. The present invention also relates to an agent for the prophylaxis or treatment of a motor nervous system or peripheral nervous system disease, which comprises, as an active ingredient, a compound having a neurotrophic factor production accelerating activity.

### Background Art

A neurotrophic factor is an endogenous factor involved in the differentiation, survival and functional maintenance of nerve cells, as well as extension of neurites depending on concentration gradient, and the like, and a glial cell line-derived neurotrophic factor (hereinafter also referred to as GDNF), a nerve growth factor (hereinafter also referred to as NGF), a brain-derived neurotrophic factor (hereinafter also referred to as BDNF), a ciliary neurotrophic factor (hereinafter also referred to as CNTF), a neurotrophin-3 (hereinafter also referred to as NT-3), an insulin-like growth factor (hereinafter also referred to as IGF), a fibroblast growth factor (hereinafter also referred to as FGF) and the like are known. The neurotrophic factor is known to protect disordered nerve cells or promote regeneration process of the nervous system after disorder. Thus, administration of a neurotrophic factor during nerve disorder is extremely effective for the prophylaxis or treatment of a motor nervous system or peripheral nervous system disorder.

However, the neurotrophic factor is a high molecular weight protein, poorly absorbed from the intestine, and cannot be expected to show a sufficient effect by oral administration. This necessitates administration via intravenous injection, etc., which places a great burden on patients. In addition, the neurotrophic factor shows poor metabolic stability in vivo, and therefore, in many cases, it is considered that sufficient effects cannot be expected. Furthermore, preparation of a neurotrophic factor in a large amount is also difficult. As described above, use of a neurotrophic factor itself for the treatment is associated with many problems, and generally difficult.

### Disclosure Of The Invention

The present inventors took note of a substance that accelerates production of a neurotrophic factor, as an alternate for a neurotrophic factor. Therefore, the present invention aims at provision of a neurotrophic factor production accelerator. In addition, the present invention aims at provision of use of a neurotrophic factor production accelerator as a medicament.

The present inventors have conducted extensive studies in view of the above-mentioned problems, and have found a substance that accelerates production of a neurotrophic factor as well as use of a substance that accelerates production of a neurotrophic factor, which resulted in the completion of the present invention. Here, as a factor whose production is to be accelerated, GDNF is particularly preferable. Accordingly, the present invention provides the following.
(1) A neurotrophic factor production accelerator comprising, as an active ingredient, a compound represented by the following formula (I): 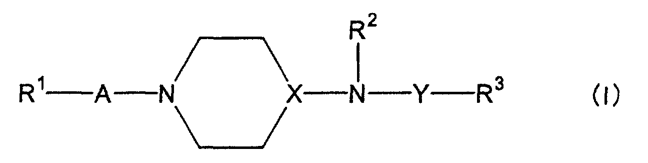 wherein R¹ is lower alkyl, aryl, ar(lower)alkoxy or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH- (hereinafter to be also referred to as Compound (I)), a salt thereof, a prodrug thereof or a solvate thereof.
(2) The accelerator of the above-mentioned (1), wherein the compound represented by the aforementioned formula (I) is N-(4-acetyl-1-piperazinyl)-p-fluorobenzamide monohydrate.
(3) A method for accelerating neurotrophic factor production, which comprises administering, to a mammal, a compound represented by the following formula (I): 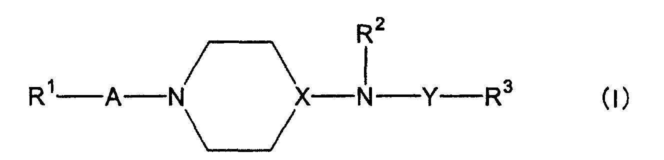 wherein R¹ is lower alkyl, aryl, ar(lower)alkoxy or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.
(4) The method of the above-mentioned (3), wherein the compound represented by the aforementioned formula (I) is N-(4-acetyl-1-piperazinyl)-p-fluorobenzamide monohydrate.
(5) Use of a compound represented by the following formula (I): 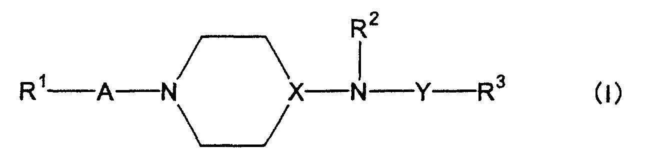 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof, for the production of a neurotrophic factor production accelerator.
(6) The use of the above-mentioned (5), wherein the compound represented by the aforementioned formula (I) is N-(4-acetyl-1-piperazinyl)-p-fluorobenzamide monohydrate.
(7) A pharmaceutical composition for accelerating neurotrophic factor production, which comprises a compound represented by the following formula (I): 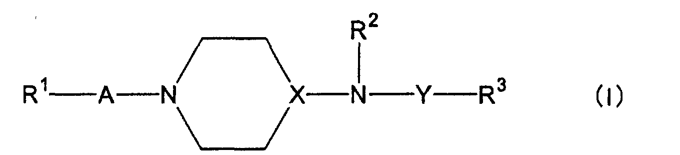 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof, and a pharmaceutically acceptable carrier.
(8) The pharmaceutical composition of the above-mentioned (7), wherein the compound represented by the aforementioned formula (I) is N-(4-acetyl-1-piperazinyl)-p-fluorobenzamide monohydrate.
(9) A commercial package comprising the pharmaceutical composition of the above-mentioned (7) or (8), and a written matter associated therewith, the written matter stating that the pharmaceutical composition can or should be used for accelerating neurotrophic factor production.
(10) An agent for the prophylaxis or treatment of a motor nervous system or peripheral nervous system disease, which comprises, as an active ingredient, a compound having a neurotrophic factor production accelerating activity.
(11) The agent of the above-mentioned (10), wherein the aforementioned motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.
(12) The agent of the above-mentioned (10) or (11), wherein the above-mentioned compound having a neurotrophic factor production accelerating activity is a compound represented by the following formula (I): 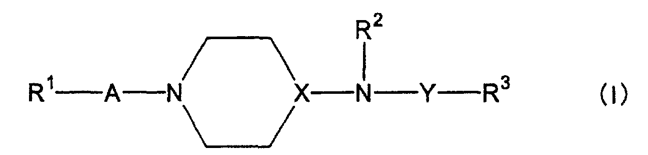 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.
(13) The agent of the above-mentioned (12), wherein the compound represented by the aforementioned formula (I) is N-(4-acetyl-1-piperaznyl)-p-fluorobenzamide monohydrate.
(14) A method of preventing or treating a motor nervous system or peripheral nervous system disease, which comprises administering a compound having a neurotrophic factor production accelerating activity to a mammal.
(15) The method of the above-mentioned (14), wherein the aforementioned motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.
(16) The method of the above-mentioned (14) or (15), wherein the aforementioned compound having a neurotrophic factor production accelerating activity is a compound represented by the following formula (I): 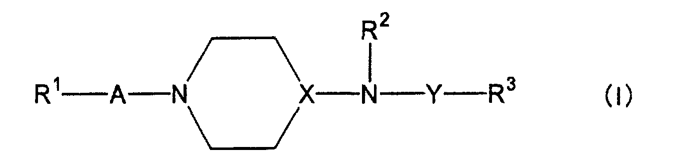 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.
(17) The method of the above-mentioned (16), wherein the compound represented by the aforementioned formula (I) is N-(4-acetyl-1-piperazinyl)-p-fluorobenzamide monohydrate.
(18) Use of a compound having a neurotrophic factor production accelerating activity for the production of an agent for the prophylaxis or treatment of a motor nervous system or peripheral nervous system disease.
(19) The use of the above-mentioned (18), wherein the aforementioned motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.
(20) The use of the above-mentioned (18) or (19), wherein the aforementioned compound having a neurotrophic factor production accelerating activity is a compound represented by the following formula (I): 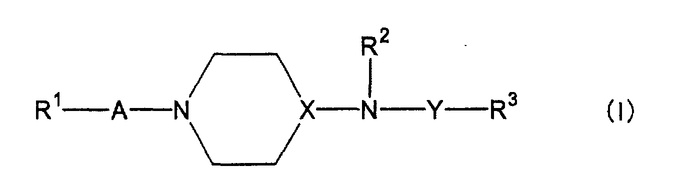 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.
(21) The use of the above-mentioned (20), wherein the compound represented by the aforementioned formula (I) is N-(4-acetyl-1-piperazinyl)-p-fluorobenzamide monohydrate.
(22) A pharmaceutical composition for the prophylaxis or treatment of a motor nervous system or peripheral nervous system disease, which comprises a compound having a neurotrophic factor production accelerating activity and a pharmaceutically acceptable carrier.
(23) The pharmaceutical composition of the above-mentioned (22), wherein the aforementioned motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.
(24) The pharmaceutical composition of the above-mentioned (22) or (23), wherein the aforementioned compound having a neurotrophic factor production accelerating activity is a compound represented by the following formula (I): 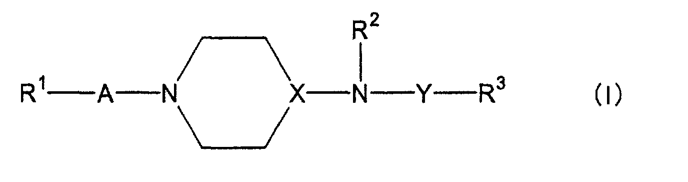 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.
(25) The pharmaceutical composition of the above-mentioned (24), wherein the compound represented by the aforementioned formula (I) is N-(4-acetyl-1-piperazinyl)-p-fluorobenzamide monohydrate.
(26) A commercial package comprising the pharmaceutical composition of any of the above-mentioned (22) to (25), and a written matter associated therewith, the written matter stating that the pharmaceutical composition can or should be used for the prophylaxis or treatment of a motor nervous system or peripheral nervous system disease.

### Brief Description Of The Drawing

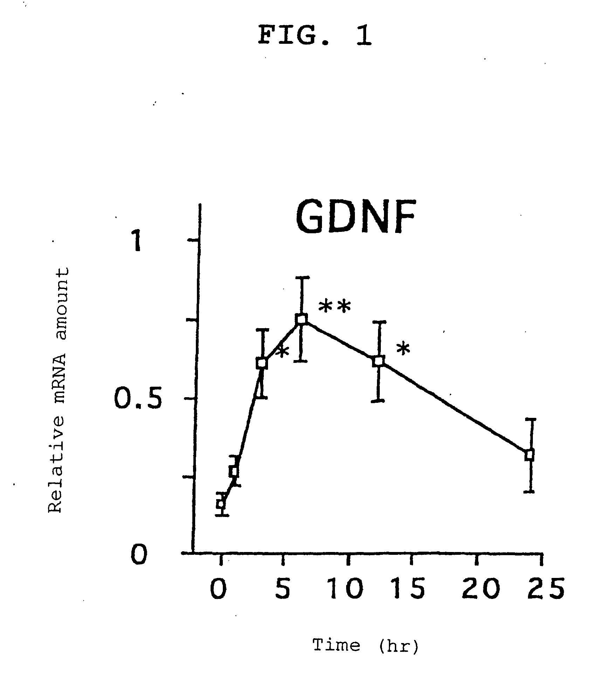

Fig. 1 is a graph showing the results of Experimental Example 1, which exhibits time-course changes in the amount of relative mRNA of GDNF after treating cultured astroglia with N-(4-acetyl-1-piperazinyl)-p-fluorobenzamide monohydrate.

### Detailed Description Of The Invention

WO00/72834 discloses that the above-mentioned Compound (I) promotes release of somatostatin in the brain and expresses long term potentiation of synaptic transmission, and therefore, it can be used as an agent for treating dementia and the like.

However, the above-mentioned publication does not disclose or suggest that Compound (I) accelerates neurotrophic factor production and has a potential of application as an agent for preventing or treating a motor nervous system or peripheral nervous system disease.

The "motor nervous system or peripheral nervous system disease" in the present invention refers to a disease caused by a disorder of motor nerves or peripheral nerves, and includes, for example, peripheral nerve disorders (neuropathy, diabetic nervous disease), myelopathy, multiple sclerosis, amyotrophic lateral sclerosis (ALS), Guillain-Barre' syndrome, Huntington's chorea, neuropathic pain and the like.

The "neurotrophic factor" in the present invention means an endogenous factor involved in the differentiation, survival and functional maintenance of nerve cells as well as extension of neurites depending on the concentration gradient and the like, and includes, for example, a glial cell line-derived neurotrophic factor (GDNF), a nerve growth factor (NGF), a brain-derived neurotrophic factor (BDNF), a ciliary neurotrophic factor (CNTF), neurotrophin-3 (NT-3), an insulin-like growth factor (IGF), a fibroblast growth factor (FGF) and the like.

The "neurotrophic factor production accelerating activity" in the present invention means an activity to accelerate production of a neurotrophic factor in vivo (significant change from the baseline). When measured according to a measurement method such as RT-PCR, for example, it means an ability to induce acceleration of a level statistically significant at a certain concentration. As a neurotrophic factor, whose production is to be accelerated by this activity, for example, GDNF, NGF, BDNF and the like as described above can be mentioned, with preference given to GDNF.

In the present invention, the "neurotrophic factor production accelerator" means a medicament having the above-mentioned activity.

The term "significant" in the above context means, in the statistics, reliability for a certain result, or conversely, a probability of such result occurring accidentally (usually 5% or less) .

Specific examples of the compound having such activity include Compound (I), a salt thereof, a prodrug thereof and a solvate thereof, with particular preference given to N-(4-acetyl-1-piperazinyl)-p-fluorobenzamide monohydrate and N-(1-acetylpiperidin-4-yl)-4-fluorobenzamide.

Each definition of the formula (I) is explained below.

"Lower" used in the present specification means a carbon number of 1 to 6 unless otherwise specified.

As the "lower alkyl", a straight or branched chain such as methyl, ethyl, propyl, isopropyl, butyl, isobutyl, tert-butyl, pentyl, hexyl and the like can be mentioned, with preference given to methyl.

As the "aryl", phenyl, naphthyl, tolyl, xylyl, mesityl, cumenyl and the like can be mentioned, with preference given to phenyl and naphthyl.

As the "ar(lower)alkoxy", benzyloxy, phenethyloxy, phenylpropoxy, benzhydryloxy, trityloxy and the like can be mentioned.

As the "heterocyclic group", a saturated or unsaturated monocyclic or polycyclic group containing at least one heteroatom (e.g., nitrogen atom, oxygen atom, sulfur atom) can be mentioned.

As suitable examples of the above-mentioned "heterocyclic group", a 3- to 8-membered, more preferably 5- or 6-membered, unsaturated heteromonocyclic group containing 1 to 4 nitrogen atom(s), such as pyrrolyl, imidazolyl, pyrazolyl, pyridyl, pyridyl N-oxide, pyrimidyl, dihydropyridyl, tetrahydropyridyl, pyrazinyl, pyridazinyl, triazinyl, triazolyl, tetrazinyl, tetrazolyl and the like; an unsaturated, condensed heterocyclic group containing 1 to 5 nitrogen atom(s), such as indolyl, isoindolyl, indolizinyl, benzimidazolyl, quinolyl, isoquinolyl, indazolyl, benzotriazolyl and the like; a 3- to 8-membered unsaturated heteromonocyclic group containing 1 or 2 oxygen atom(s) and 1 to 3 nitrogen atom(s), such as oxazolyl, isoxazolyl, oxadiazolyl and the like; a 3- to 8-membered saturated heteromonocyclic group containing 1 or 2 oxygen atom(s) and 1 to 3 nitrogen atom(s), such as morpholino, sydnonyl and the like; an unsaturated, condensed heterocyclic group containing 1 or 2 oxygen atom(s) and 1 to 3 nitrogen atom(s), such as benzoxazolyl, benzoxadiazolyl and the like; a 3- to 8-membered unsaturated heteromonocyclic group containing 1 or 2 sulfur atom(s) and 1 to 3 nitrogen atom(s), such as thiazolyl, isothiazolyl, thiadiazolyl and the like; a 3- to 8-membered unsaturated heteromonocyclic group containing 1 or 2 sulfur atom(s), such as thienyl and the like; an unsaturated, condensed heterocyclic group containing 1 or 2 sulfur atom(s) and 1 to 3 nitrogen atom(s), such as benzothiazolyl, benzothiadiazolyl and the like; a 3- to 8-membered unsaturated heteromonocyclic group containing one oxygen atom, such as furyl and the like; an unsaturated, condensed heterocyclic group containing 1 or 2 sulfur atom(s), such as benzothienyl and the like; and an unsaturated, condensed heterocyclic group containing 1 or 2 oxygen atom(s), such as benzofuranyl and the like can be mentioned.

"Cyclo(lower)alkyl" is a cycloalkyl group having a carbon number of 3 to 6, such as cyclopropyl, cyclobutyl, cyclopentyl and the like.

"Ar(lower)alkyl" includes benzyl, phenethyl, phenylpropyl, benzhydryl, trityl and the like.

As the "lower alkylene", methylene, ethylene, propylene, pentamethylene, hexamethylene and the like can be mentioned.

The aforementioned lower alkyl, aryl, ar(lower)alkoxy, heterocyclic group, cyclo(lower)alkyl and ar(lower)alkyl may be substituted by 1 to 6 halogen atom(s) (e.g., fluorine, chlorine, bromine and iodine).

In the present invention, the "agent for the prophylaxis or treatment of a motor nervous system or peripheral nervous system disease" is a medicament containing a compound having the above-mentioned accelerating activity as an active ingredient. While the motor nervous system or peripheral nervous system disease to be prevented or treated is not particularly limited as long as its symptoms can be prevented or alleviated as a result of acceleration of the neurotrophic factor production, the medicament of the present invention is particularly effective for the prophylaxis or treatment of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.

The neurotrophic factor production accelerator and the agent for the prophylaxis or treatment of a motor nervous system or peripheral nervous system disease, which contains, as an active ingredient, a compound having a neurotrophic factor production accelerating activity of the present invention (hereinafter the accelerator and the prophylactic or therapeutic agent are also collectively referred to as a medicament of the present invention), can be administered in the dosage form of a solid, semi-solid or liquid preparation containing an organic or inorganic carrier or excipient suitable for rectal administration, inhalation, nasal drop, eye drop, external (local), oral or parenteral administration (including subcutaneous, intravenous and intramuscular administrations), direct administration to the lesion such as brain, spinal fluid, cerebroventricle and the like, or inhalation.

The medicament of the present invention can be combined with a pharmaceutically acceptable, substantially non-toxic carrier or excipient conventionally used for the dosage forms such as tablet, pellets, troche, capsule, suppository, cream, ointment, aerosol, inhalation powder, liquid, emulsion, suspension, and other dosage forms suitable for use. Where necessary, an auxiliary agent, a stabilizer, a tackifier, a coloring agent and a flavor can be added.

The medicament of the present invention can be produced by a preparation technique well known in the pertinent field. A compound having a neurotrophic factor production accelerating activity in the present invention can be prepared into the form of a salt thereof, a prodrug thereof or a solvate thereof, as necessary, using a method known in the pertinent field. The medicament of the present invention can also be prepared using such salt, prodrug or solvate of the compound of the present invention.

"The salt" in the present invention is preferably a biologically acceptable, generally non-toxic salt, which is exemplified by acid-addition salts such as an inorganic acid addition salt (e.g., hydrochloride, hydrobromide, sulfate, phosphate and the like), acid-addition salts of organic carboxylic acid or a sulfonic acid (e.g., formate, acetate, trifluoroacetate, maleate, tartrate, fumarate, methanesulfonate, benzenesulfonate, toluenesulfonate and the like), salts with acidic amino acid (e.g., aspartic acid, glutamic acid and the like).

"The prodrug" in the present invention is preferably a compound that is converted to a compound having a neurotrophic factor production accelerating activity based on a reaction with a gastric acid or an enzyme in the body.

"The solvate" in the present invention is, for example, an inclusion compound (e.g., hydrate).

When the medicament of the present invention is applied to mammals (including human), it is preferably administered intravenously (including a method comprising adding the medicament to infusion), intramuscularly or orally.

The medicament of the present invention only needs to be contained in a preparation at least in an amount sufficient to provide a desired effect in the progression or condition of the target disease.

While the dose and administration route of the medicament of the present invention vary depending on the kind of compound, age and conditions of the patients under prophylaxis and/or treatment, daily dose is, for example, 0.01 to 10 mg/kg body weight on the basis of the amount of the active ingredient Compound (I), by oral administration, which may be administered once a day or in a few portions a day for the prophylaxis or treatment of the aforementioned diseases.

### Examples

The present invention is explained in more detail in the following by referring to Examples, which are mere examples of preferable embodiments of the present invention, and do no limit the invention in any way.

### Example 1

### Preparation of cultured astroglia

Astroglia was prepared from the brain of a 1 to 2-day-old Wistar rat. It was inoculated to a 75 cm² culture flask, and incubated in Eagle's minimum essential medium supplemented with 10% fetal bovine serum for 10 to 14 days. Then, it was shaken overnight at 250 rpm, and microglia and oligodendroglia on the astroglia monolayer were removed. Astroglia was dispersed in 0.25% trypsin (1: 400, Invitrogen), and inoculated to a 6-well plate. It was further incubated for 7 to 10 days, and confluent cells were used for the experiment.

### Quantification of mRNA by RT-PCR

The cultured astroglia prepared by the above-mentioned method was incubated in serum-free Eagle's minimum essential medium for 48 hours. The total RNA was prepared by AGPC (Acid Guanidinium-Phenol-Chloroform) method (Chomczynski P., and Sacchi N., Anal. Biochem. 162, 156-159 (1987)). RNA (1 µg) was then subjected to a reverse transcription reaction, and cDNA fragment of GDNF was amplified by PCR using the primers shown below. After completion of the reaction, the reaction product amplified by PCR was electrophoresed on a 1.5% agarose gel in TBE buffer (90 mM tris-borate-2 mM EDTA), and incubated in TBE buffer containing 0.01% Vistra Green (Biotech) for 30 minutes. The fluorescence intensity of the PCR product was imaged by Fluoro Imager (Molecular Dynamics), and fluorescence bands were quantified by NIH Image. The amount of the PCR product of the neurotrophic factor was standardized using the amount of the β-actin PCR product of cDNA, and the amount of relative mRNA was determined. The activity of N-(4-acetyl-1-piperazinyl)-p-fluorobenzamide monohydrate (hereinafter to be also referred to as a test drug.) on neurotrophic factor production was evaluated by a Tukey-Kramer assay.

### <Primer used>

· β-actin 
upper: 5'-gat ggt ggg tat ggg tca gaa gga-3' (SEQ ID NO. 1)
lower: 5'-gct cat tgc cga tag tga tga cct-3' (SEQ ID NO. 2)
· GDNF

upper: 5'-atgaag ttatgg gatgtc gt-3' (SEQ ID NO. 3)
lower: 5'-cagggt cagata catcca ca-3' (SEQ ID NO. 4)

The primer of β-actin used as described above was prepared according to Martres MP. *et al.,* J. Neurochem., 58, 673-679 (1992). In addition, the GDNF primer used was prepared according to Appel E. *et al.,* Neuroreport. 8, 3309-3312 (1997).

### <Results>

Fig. 1 shows time-course changes of relative mRNA amount of the neurotrophic factor standardized using the amount of the β-actin PCR product. The amount of GDNF mRNA showed significant increase from 3 hours after the treatment with the test drug, and showed changes having a peak 6 hours later.

From these results, it was shown that the test drug had a neurotrophic factor production accelerating activity in vivo.

### Sequence Listing Free Text

SEQ ID NO. 1: Oligonucleotide designed to act as a PCR primer for detecting β-actin gene.

SEQ ID NO. 2: Oligonucleotide designed to act as a PCR primer for detecting β-actin gene.

SEQ ID NO. 3: Oligonucleotide designed to act as a PCR primer to detect a glial cell line-derived neurotrophic factor (GDNF) gene.

SEQ ID NO. 4: Oligonucleotide designed to act as a PCR primer to detect a glial cell line-derived neurotrophic factor (GDNF) gene.

### Industrial Applicability

The present invention provides a neurotrophic factor production accelerator, and further a neurotrophic factor production accelerator useful for the prophylaxis or treatment of a motor nervous system or peripheral nervous system disease such as peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.

This application is based on a patent application No. 108552/2002 filed in Japan, the contents of which are hereby incorporated by reference.

## Claims

1. A neurotrophic factor production accelerator comprising, as an active ingredient, a compound represented by the following formula (I): 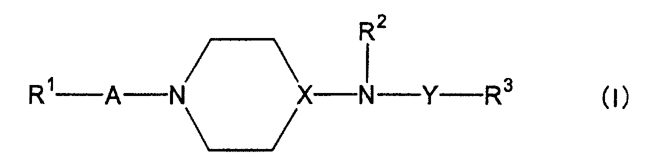 wherein R¹ is lower alkyl, aryl, ar(lower)alkoxy or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.

2. The accelerator of claim 1, wherein the compound represented by the formula (I) is N-(4-acetyl-1-piperazinyl)-p-fluorobenzamide monohydrate.

3. A method for accelerating neurotrophic factor production, which comprises administering, to a mammal, a compound represented by the following formula (I): 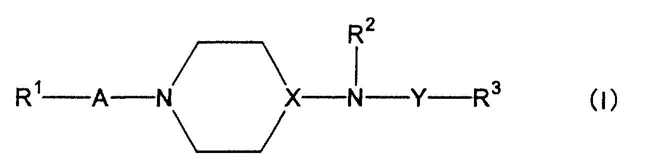 wherein R¹ is lower alkyl, aryl, ar(lower)alkoxy or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.

4. The method of claim 3, wherein the compound represented by the formula (I) is N-(4-acetyl-1-piperazinyl)-p-fluorobenzamide monohydrate.

5. Use of a compound represented by the following formula (I): 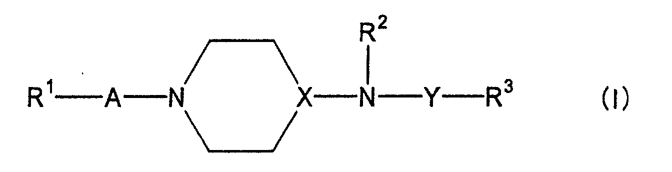 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof, for the production of a neurotrophic factor production accelerator.

6. The use of claim 5, wherein the compound represented by the formula (I) is N-(4-acetyl-1-piperazinyl)-p-fluorobenzamide monohydrate.

7. A pharmaceutical composition for accelerating neurotrophic factor production, which comprises a compound represented by the following formula (I): 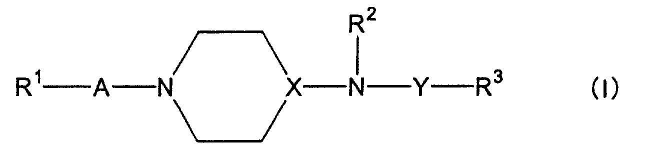 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof, and a pharmaceutically acceptable carrier.

8. The pharmaceutical composition of claim 7, wherein the compound represented by the formula (I) is N-(4-acetyl-1-piperazinyl)-p-fluorobenzamide monohydrate.

9. A commercial package comprising the pharmaceutical composition of claim 7 or 8, and a written matter associated therewith, the written matter stating that the pharmaceutical composition can or should be used for accelerating neurotrophic factor production.

10. An agent for the prophylaxis or treatment of a motor nervous system or peripheral nervous system disease, which comprises, as an active ingredient, a compound having a neurotrophic factor production accelerating activity.

11. The agent of claim 10, wherein the motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.

12. The agent of claim 10 or 11, wherein the compound having a neurotrophic factor production accelerating activity is a compound represented by the following formula (I): 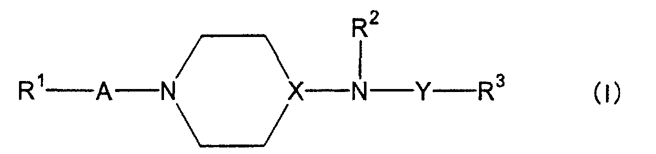 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.

13. The agent of claim 12, wherein the compound represented by the formula (I) is N-(4-acetyl-1-piperaznyl)-p-fluorobenzamide monohydrate.

14. A method of preventing or treating a motor nervous system or peripheral nervous system disease, which comprises administering a compound having a neurotrophic factor production accelerating activity to a mammal.

15. The method of claim 14, wherein the motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.

16. The method of claim 14 or 15, wherein the compound having a neurotrophic factor production accelerating activity is a compound represented by the following formula (I): 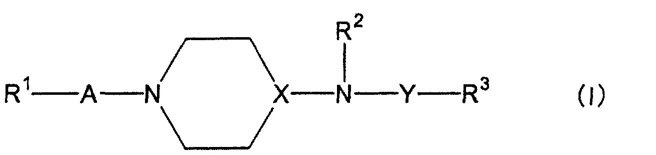 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.

17. The method of claim 16, wherein the compound represented by the formula (I) is N-(4-acetyl-1-piperazinyl)-p-fluorobenzamide monohydrate.

18. Use of a compound having a neurotrophic factor production accelerating activity for the production of an agent for the prophylaxis or treatment of a motor nervous system or peripheral nervous system disease.

19. The use of claim 18, wherein the motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.

20. The use of claim 18 or 19, wherein the compound having a neurotrophic factor production accelerating activity is a compound represented by the following formula (I): 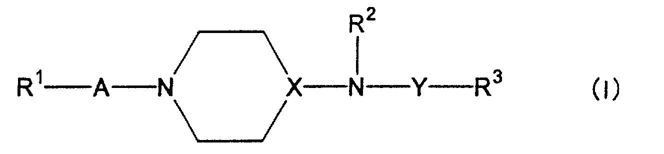 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.

21. The use of claim 20, wherein the compound represented by the formula (I) is N-(4-acetyl-1-piperazinyl)-p-fluorobenzamide monohydrate.

22. A pharmaceutical composition for the prophylaxis or treatment of a motor nervous system or peripheral nervous system disease, which comprises a compound having a neurotrophic factor production accelerating activity and a pharmaceutically acceptable carrier.

23. The pharmaceutical composition of claim 22, wherein the motor nervous system or peripheral nervous system disease is selected from the group consisting of a peripheral nerve disorder (neuropathy, diabetic nervous disease), myelopathy, multiple sclerosis, amyotrophic lateral sclerosis (ALS), Guillain-Barre' syndrome, Huntington's chorea and neuropathic pain.

24. The pharmaceutical composition of claim 22 or 23, wherein the compound having a neurotrophic factor production accelerating activity is a compound represented by the following formula (I): 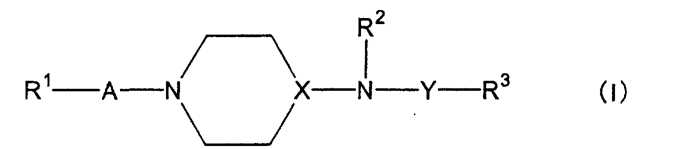 wherein R¹ is lower alkyl, aryl, ar(lower)alkoxy, or a heterocyclic group, the above groups being optionally substituted by halogen, R² is a hydrogen atom or lower alkyl, R³ is cyclo(lower)alkyl, aryl or ar(lower)alkyl, the above groups being optionally substituted by halogen, A is -CO-, -SO₂- or lower alkylene, X is N or CH, and Y is -CO-, -SO₂- or -CONH-, a salt thereof, a prodrug thereof or a solvate thereof.

25. The pharmaceutical composition of claim 24, wherein the compound represented by the formula (I) is N-(4-acetyl-1-piperazinyl)-p-fluorobenzamide monohydrate.

26. A commercial package comprising the pharmaceutical composition of any of claims 22 to 25, and a written matter associated therewith, the written matter stating that the pharmaceutical composition can or should be used for the prophylaxis or treatment of a motor nervous system or peripheral nervous system disease.
